# EUROPEAN PATENT APPLICATION

(11) **EP 3 388 054 A1**
(43) Date of publication of application: **17.10.2018**
(21) Application number: 17000630.8
(22) Date of filing: 12.04.2017
(51) Int. Cl.: A61K 9/107, A61K 47/10, A61K 47/14, A61K 47/24, A61K 47/34, A61K 47/38, A61K 47/40, A61K 9/127, A61K 9/14

(54) **DISPENSING CAP CONTAINING A SOLUBILISATE OF A PHARMACEUTICALLY ACTIVE AGENT OR DIETARY SUPPLEMENT**

(71) Applicant: Athenion AG, 6304 Zug (CH)
(72) Inventor: The designation of the inventor has not yet been filed

(57) **Abstract**

The present invention relates to a dispensing cap for the delivery of a solubilisate, a beverage system configured for the use of such a dispensing cap and a method for preparing a beverage by means of such a beverage system. Poorly water-soluble dietary supplements or pharmaceutical active agents can be delivered in this new dosage form in order to increase the bioavailability of these substances.

## Description

The present patent application refers to a dispensing cap containing a solubilisate of a pharmaceutically active agent or dietary supplement. Suitable dispensing caps and technologies for producing such a solubilisate are disclosed. Moreover, the present patent application refers to a beverage system containing such a dispensing cap.

A broad variety of substances are known for which potentially beneficial effects for the human health have been found in *in vitro* experiments. The use of many of them, however, has been seriously limited by the poor bioavailability that can be achieved by application forms known in the state-of-the-art. In pharmacology, bioavailability is a parameter that indicates the fraction of an administered dose of unchanged drug that finally becomes available in the systemic circulation and thus may provide the desired pharmacological effects. This poor bioavailability is often due to a poor water solubility, respectively the lipophilic nature of the active agent to be administered. Hence the use of such substances as a pharmaceutically active agent or dietary supplement is impaired when using standard dosage forms.

There is a variety of approaches for improving the solubility of such agents and in many cases also their bioavailability by using specific solubilization techniques. Herein the solubility of an agent in a medium is augmented by adding a third substance. These third substances are referred to as solubilizers (solubilizing agents), substances that may for example build a complex with the substance to be solubilized. Examples for such chelating agents are sodium benzoate and sodium salicylate. Another mechanism of action of solubilizers is the augmentation of the dissolving capacity of the solvent, for example by disturbing the cluster structure of water. Examples for such structure breakers are glycerol (glycerin) and macrogols (polyethylene glycol, PEG).

A third solubilization mechanism are micelle and liposome application technologies. They gained broad attention throughout the last decades. Herein the substance to be delivered is enclosed in a spherical aggregate of surfactant molecules. These molecules are characterized by a polar head group and a long nonpolar chain ("tail"). When given into an aqueous medium these molecules tend to associate by aggregating to spherical structures by orienting the polar head group towards the surrounding medium and the nonpolar chain towards the interior of the spheres. When these spheres consist of only one layer of such amphiphilic molecules they are referred to as micelles. Depending on the nature of the amphiphilic molecule and the reaction conditions it is also possible to form spheres with more than one layer. Herein a second layer is formed inside the outer layer of the sphere, the nonpolar groups of this second layer being oriented towards the nonpolar groups of the outer layer, and the polar head groups being oriented towards the interior of the sphere. Such aggregates are referred to as liposomes. In their structure they resemble the lipid bilayer of the cell membrane. Often the same or structurally related components are used for liposomes as those known from the cell membrane, therefore displaying similar physicochemical properties. There are also multi-layered liposomes in which at least two liposomal spheres are formed over one another, thus building a multispherical aggregate. When given in a lipophilic medium these substances tend to build inversed spherical structures where the lipophilic chain is oriented towards the solution medium and the other layers are arranged accordingly.

It is known for a long time that it is possible to enclose substances inside such spherical structures. Different uses of such loaded spheres have been described in the art, among them the usage as a dosage form for the application of lipophilic substances and/or for increasing the bioavailability of the enclosed substance. In micelles, the enclosed nonpolar substance concentrates in the interior space of the sphere toward which the nonpolar chains of the amphiphilic molecules are oriented. In liposomes, however, the interior space of the spheres is an aqueous, respectively hydrophilic medium. It can serve for packaging hydrophilic molecules. Poorly water-soluble, respectively lipophilic molecules, however, gather mostly in between the lipophilic structures of the liposomal layers.

From empiric pharmacokinetic measurements it is known that micelles as well as liposomes are absorbed from the organism in the gastrointestinal tract to a comparatively high degree, in particular from the intestinal villi. Thus a substance packed in such a spherical aggregate is absorbed to a much higher degree into the systemic bloodstream into which a certain percentage of the enclosed substance is released through different physiologic and non-physiologic mechanisms. Thus this substance becomes bioavailable and can exert its actions in the organism. If needed it can be transported via the cellular membrane to the interior of a cell. The transport, respectively the absorption rate over the cell membrane is an intrinsic feature of each substance, depending on a variety of factors such as molecule size, degree of lipophilicity and the presence of suitable transporter molecules inside the cell membrane. In general, lipophilic substances are transported more easily over the cell membrane because of the lipophilic nature of the lipid bilayer of the cell membrane. From liposomes it is also known that they are able to fuse with the cell membrane by invagination, thus delivering the enclosed substance to a considerable degree into the cytosol. Certain cell types, in particular phagocytes such as macrophages, monocytes and neutrophils, preferably ingest liposomes which then may undergo metabolic digestion and thus deliver the enclosed substance to these cells.

Liposomal applications have been widely discussed in medicine and pharmacology and many sophisticated technologies for their production have been developed. Their use, however, is not very common. One reason are the relatively high production costs, another reason are possible adverse side effects. In particular, when parenterally applied, liposomes carry the risk of accumulating in the liver, the spleen and/or the bone marrow. This problem rather seldom occurs in oral dosage forms.

Self-emulsifying drug delivery systems (SEDDS) are another approach to solubilize lipophilic compounds. They use to be isotropic mixtures of oils, surfactants, solvents and optionally cosolvents. Depending on the used components they may form solubilisates or stable oil-in-water (o/w) emulsions upon aqueous dilution and optionally gentle agitation.

Another solubilization technique is the formation of inclusion complexes of the substance to be solubilized with cyclodextrins such as α-, β- or γ-cyclodextrin or cyclodextrin derivatives such as 2-hydroxypropyl-β-cyclodextrin, methyl-β-cyclodextrin or trimethyl-β-cyclodextrin. Typically, cyclodextrins are composed of 6 to 8 1,4-linked α-D-glucopyranosides forming macrocycles. Thus a water-soluble toroid (respectively cone-shaped or bucket-shaped) structure is generated which is capable to host hydrophobic substances in its interior. The interior space is considerably less hydrophilic than the outside contacting the aqueous environment. Cyclodextrins are produced from starch by enzymatic treatment. They are loaded with the compound to be solubilized by dispersion. The compound to be solubilized can then be released by contacting these complexes with water, by pH or temperature changes, depending on the specific composition. Cyclodextrins are used a.o. for dietary supplements (e.g. Cavamax^{®} by Wacker Chemie, Germany) or for pharmaceutical drug delivery (e.g. for diclofenac (EP 0 658 347 A2) or clarithromycin (Allsopp, (2013), Development of a soluble macrolide formulation and identification of potential benefits in chronic rhinosinusitis, MPhil Thesis, University of Queensland). If recrystallization of the compound to be solubilized occurs above a certain final concentration often an emulsifier such as a polysorbate (e.g. Tween^{®} 20; EP 1 609 481 A1) is added.

Examples for solubilized dietary supplements and/or pharmaceutically active agents are disclosed in US 2013/150396 A1, EP 2468111 A1 and US 2003/091627 A1.

Often it is not very comfortable to mix such solubilisates with the diluents. It is often regarded as cumbersome and weary, in particular when the mixing takes time, or stirring or agitation of the final beverage is required. A major problem is the appeal of such a procedure, in particular when the beverage is not prepared by medical staff but by the consumer or the patient himself. Consumers strongly prefer quick preparation methods and an easy handling. For example, opening an ampule oneself, although relatively safe nowadays, is a strong emotional obstacle for many consumers and patients. In case of a medication this may often lead to a poor patient compliance causing a wake of consequential problems. Further, the mixing procedure is often insufficient and may result in a poorly solubilized beverage and/or an inhomogeneous beverage, respectively liquid dosage form. Another aspect is that the solubilization process often leads to a phenomenon called zebra effect or striping caused by an incomplete dissolution of the solubilisate (or other concentrate). Such a beverage is not particularly appealing for intake.

Therefore there is a need for new, respectively alternative dosage forms of such solubilized substances for use as a dietary supplement or pharmaceutically active agent that overcome the problems mentioned above.

Thus it is the task of this patent application to provide a consumer-friendly easy-to-handle and safe liquid oral dosage form for dietary supplements or pharmaceutically active agents in need to be solubilized.

### Description of the invention

Surprisingly, it was found that dispensing caps containing such a solubilisate are able to solve these problems.

Thus the present invention refers to a dispensing cap containing a solubilisate of at least one pharmaceutically active agent and/or a dietary supplement.

The present invention discloses a dispensing cap with at least one fillable chamber containing a solubilisate of at least one pharmaceutically active agent and/or dietary supplement, wherein the dispensing cap is suitably configured to be mounted on an outlet of a beverage container and said solubilisate can be released into the beverage container by operating a releasing mechanism of the dispensing cap.

In particular, the present invention refers to a dispensing cap containing a solubilisate of at least one pharmaceutically active agent and/or a dietary supplement, wherein the solubilisate is prepared from at least one poorly water-soluble substance or extract.

It is preferred that said solubilisate is prepared by means of micelle, liposome, self-emulsification or cyclodextrin complexation technology.

In case of self-emulsification the technology disclosed in EP 16001941.0 is preferred.

Dispensing caps are closures of a beverage container that are apt to release their content into the liquid inside the beverage container. They feature a chamber for storing the content to be released. In a common embodiment, a plunger on the surface of the dispensing cap has to be pressed down by the consumer. Thus the plunger drives directly a sharp tool which pierces a membrane separating this storage chamber from the liquid inside the beverage container, thus allowing the content of the chamber to be released into the drink. Some embodiments of dispensing caps combine the separated storage option with the drinking comfort of a sports drink. Herein the so-called push-pull mechanism is combined with the dispensing cap. This mechanism is also known as sports cap system. After pushing down said plunger a combined part of the dispensing cap is pulled back and turns into a spout enabling controlled drinking from the bottle and reclosure thereon. Advanced dispensing caps may include more than one chamber for storing different substances to be released. After the release into the drinkable liquid these substances become at least partially solubilized. Thus it can be prevented that oxidation in the aqueous environment, light or heat degradation is to occur prematurely.

However, a fresh preparation does not automatically ensure a high bioavailability. The desired effect is often hampered by the limited absorption rate of the respective pharmaceutically active agent or dietary supplement during its passage through the intestinal tract. Either a considerable percentage of a specific substance passes through without being absorbed, or absorption takes place over an extended period of time so that high physiologically effective first peak concentrations of a specific supplement can't be achieved. The setting becomes even more complex when several substances are involved that are aimed to peak at the same time, either for seeking a complementary effect, or an additive mode of action.

Therefore it is the task of the present patent application to provide a fresh preparation of a poorly water-soluble pharmaceutically active agent or dietary supplement in a drinkable liquid. Ideally, this dosage form leads to a significantly enhanced bioavailability of this substance.

Surprisingly, it was found that this task can be solved by a dispensing cap according to the invention. Further advantageous embodiments are described in the following and in the dependent claims.

The dispensing cap according to the invention must have at least one chamber that can be filled with a solubilisate of at least one pharmaceutically active agent or dietary supplement. This at least one chamber is by default separated from the interior of the beverage container into which said solubilisate shall be released. There are two major principles through which said solubilisate can be released from the at least one chamber of the dispensing cap into the drinkable liquid inside the beverage container.

First, a moveable part of the dispensing cap is operated by the releasing mechanism of the dispensing cap. It is shifted in such a manner that it gives way for releasing the at least one supplement. This can be for example a folding mechanism, a tilting mechanism, a trap door mechanism, a pivot lock mechanism, a diaphragm (aperture) mechanism, a conveyor mechanism, a screw conveyor mechanism. On being triggered such a mechanism can allow a temporally and/or spatially limited release or an unlimited release of the at least one supplement. The advantage of such a releasing mechanism is the option for a refill of the at least one chamber of the dispensing cap with the same or another solubilisate.

The second separation type comprises at least one membrane, diaphragm, film or seal foil that is fully spanned between the at least one chamber of the dispensing cap and the interior of the beverage container, thus completely separating these two compartments. This at least one membrane, diaphragm, film or seal foil is sufficiently sustainable for carrying the weight of the solubilisate without being ruptured prematurely. Also it has to endure normal transport movements of the container or mild to medium vibrations, concussions or agitations, for example when being used as part of a sports drink. For said release of the solubilisate this membrane, diaphragm, film or seal foil is punched, pierced or cut by a sharp cutting tool, respectively a puncher. This way an opening is generated through the membrane, diaphragm, film or seal foil that allows either a temporally and/or spatially controlled release of the solubilisate or a complete release at once. The advantage of these foils is a better isolation of the solubilisate from oxygen, moisture, light, UV irradiation and/or heat.

In preferred embodiments a combination of the at least one membrane, diaphragm, film or seal foil and cutting tool is chosen that ensures that virtually no debris gets into the drinkable liquid of the beverage container or contaminates the solubilisate to be released.

In another preferred embodiment the at least one membrane, diaphragm, film or seal foil is pre-punctured, thus enabling, respectively facilitating a controlled cutting line on operating said cutting tool.

This at least one membrane, diaphragm, film or seal foil can be made of metal (such as aluminium foil, tin foil, silver foil, gold foil, copper foil), a polymer (such as polyolefins, polyethylene, polypropylene, polyvinylchloride, polystyrene, polycarbonate, cellophane, cellulose esters such as cellulose acetate and nitrocellulose, polylactic acid, polyester, in particular polyhydroxyalkanoates such as poly-3-hydroxybutyrate, polyhydroxyvalerate and polyhydroxyhexanoate, polyamide 11, polyethylene terephthalate, starch blends, parafilm), paper (such as writing paper, rolling paper, banana paper, waterproof paper, parchment paper, greaseproof paper, wax paper, cardboard, wrapping tissue), a rubber stopper (such as canonized in current pharmacopoeias) or a composite material produced from the aforementioned materials (such as blisters).

In preferred embodiments the thickness of a metal foil is less than 20 µm, more preferred less than 18 µm, most preferred less than 15 µm.

In preferred embodiments the ultimate tensile strength of a metal foil is in the range of 20 - 220 N/mm², more preferred between 30 - 160 N/mm², even more preferred between 40 - 125 N/mm², most preferred between 45 - 95 N/mm².

In preferred embodiments the thickness of a polymer-based foil is less than 100 µm, more preferred less than 80 µm, most preferred less than 50 µm.

In preferred embodiments the ultimate tensile strength of a polymer-based foil is in the range of 20 - 300 N/mm², more preferred between 40 - 250 N/mm², even more preferred between 60 - 200 N/mm², most preferred between 80 - 180 N/mm².

In preferred embodiments the thickness of said paper is less than 200 µm, more preferred less than 160 µm, most preferred less than 120 µm.

In preferred embodiments the bursting strength of said paper is in the range of 100 - 700 KPa, more preferred between 150 - 500 KPa, even more preferred between 200 - 400 KPa, most preferred between 250 - 300 KPa.

For embodiments in need of a particularly tight-sealing foil (for example for excluding oxygen entry to oxidation-sensitive supplements such as vitamin C) metal foils are preferred.

One problem of the use of foil materials is the degradation after use, particularly in the environment. Many of these materials are very poorly degradable under natural conditions and may contribute over years to the environmental load. Moreover, many of the plastic-based materials are derived from petroleum. In the light of resource scarcity and the power consumption needed for their production this is not always desirable. Therefore in preferred embodiments non-toxic biodegradable materials are used. Moreover, their energy balance is more favorable. Such preferred foil materials are cellulose acetate, nitrocellulose, polylactic acid, poly-3-hydroxybutyrate, polyhydroxyvalerate, polyhydroxyhexanoate, polyamide 11, starch blends.

Another problem is that on cutting or punching the foil often it can't be completely avoided that tiny debris of the foil gets into the liquid in the beverage container and thus is likely to be imbibed by the consumer. Though there are no long-term studies about health hazards of such foil debris it is certainly preferable to avoid or minimize such risks by a) using cutting tools that generate only minimal amounts of debris on being used, b) the use of biodegradable materials that can be relatively quickly degraded by the organism, either by gastric acid or by aerobic or anaerobic bacteria in the intestinal tract (see above), or c) by biocompatible materials that will pass unchanged through the intestinal tract and don't accumulate in the organism.

A preferred example for such a biocompatible foil material is polypropylene.

The at least one membrane, diaphragm, film or seal foil can be punched, pierced or cut as a whole or compartment-, respectively sector-wise.

The cutting tool can have the shape of a puncher, a stamp, a blade, a knife, a cutting disc, a scissor, a milling cutter, a drill, a chisel. It can be driven vertically or in a beveled angle through the material to be cut. It can be made of metals, medical stainless steel, alloys, rigid plastics, glass, ceramics, diamond, boron nitride.

This at least one chamber can be simply the interior space of the dispensing cap, enclosed by the still intact seal foil.

However, there can be a more compact compartmentalization inside the dispensing cap, creating an enclosed space for one or more fillable chambers. The wall architecture can vary according the specific needs of the solubilisate to be stored therein. It is preferred that the material for these compartment walls is the same material as the dispensing cap. So the entire dispensing cap could be produced by injection molding techniques from thermoplastics.

In the state-of-the-art a variety of dispensing caps are known that fulfill the requirements laid out above or can be adapted in such a manner. This group comprises a.o. ViCap^{®}, VizCap, Activate^{®} cap, Optima functional cap, BioGaia Cap, Cedevita cap, INCAP, PowerCap^{®}, Yoli Blast Cap, Mojo organics cap, Karma cap^{®}, Tap-the-Cap^{®}, Delo Cap^{®}, Aspin^{®} Dispensing Bottle Cap, CapStaticX, Berocca Twist 'N' Go^{®}, Fusion Cap. A comprehensive overview over the respective modes of action and producers, additionally discussing the respective advantages and disadvantages, can be found in Anton Steeman's tripartite article series from 2012 Developments in Dispensing Caps - An Overview on http://bestinpackaginct.com (as of Feb 6th, 2017).

Thus according to the invention any of these dispensing cap types can be used. The use of INCAP is preferred.

In several embodiments the dispensing cap has additionally a removable protective cap in order to protect the releasing mechanism of the dispensing cap, for example from mechanical damage during transport, storage or handling, or from involuntary premature activation of the release mechanism by a careless user. It can also serve as a dust cover.

Such a protective cap should be easily removable. It can be either pulled off from the proper dispensing cap as a separate piece. There can be an integrated flip-flop cap mechanism (the protective cap is articulated with the proper dispensing cap through flexible plastic strips) or a tear strip mechanism (a strip has to be removed from the protective cap. Thus the protective cap is easily removable).

The first two types feature the advantage of being replaceable on the proper dispensing cap. The tear strip mechanism has the advantage that it is particularly tight sealing and it ensures that the beverage system, respectively the dispensing cap hasn't been used before. This can be desirable for dispensing caps storing particular valuable supplements such as pharmaceutically active agents.

Hence a protective cap with a tear strip mechanism is preferred.

According to the invention it is also possible to store different solubilisates in separate chambers of the dispensing cap. This can be advantageous when said solubilisates are likely to interact in an unwanted manner which could deteriorate the effective content and thus finally bias the bioavailability of the substances included in said solubilisates. The solubilisates from these separated chambers may be released concomitantly or consecutively into the drinking liquid. They may be sealed off from the container by one foil for the entire dispensing caps or with an individual foil for each chamber. The latter variant allows for a release at staggered intervals. So the user may decide individually which amount, respectively which solubilisate or solubilisate combination should be released at a certain time. This increases the user comfort by offering him more degrees of freedom in his choice which may show to be advantageous for example for the attractiveness the product. It may also allow for a more individualized medication of combination drugs. Such a freely eligible staggered release can be carried out either via individual cutting tools for each chamber, or via one cutting tool that can be individually assigned to a specific chamber by the user. In embodiments with a quantitatively staggered release option this can be achieved by a rotatory motion control of the cutting tool, thus cutting open the consecutive chambers one by one. The user may intuitively track the advance of the cutting tool by a slight snap, respectively a click sound.

The dispensing caps as described before are apt to host a broad variety of solubilisates. Therefore they are suitable for many different areas of application, ranging from standard dietary supplements over lifestyle products to innovative pharmaceutical dosage forms.

According to the current United States Food and Drug Administration (FDA) definition, the term dietary supplements refers to substances or products which are not pharmaceutical drugs, food additives like spices or preservatives, or conventional food and also meet any of the following criteria:
1) The product is intended to supplement a person's diet, despite it not being usable as a meal replacement.
2) The product is or contains a vitamin, dietary element, herb used for herbalism or botanical used as a medicinal plant, amino acid, any substance which contributes to other food eaten, or any concentrate, metabolite, ingredient, extract, or combination of these things.
3) The product is labeled as a dietary supplement.

Herbal products are made from one or more herbs. If more than one herb is used, the term mixture herbal product can also be used. Finished herbal products and mixture herbal products may contain excipients in addition to the active ingredients. However, finished products or mixture products to which chemically defined active substances have been added, including synthetic compounds and/or isolated constituents from herbal materials, are not considered to be herbal.

As a pharmaceutically active agent or a dietary supplement may be sensitive to oxidation processes, in another embodiment according to the invention the solubilization process and/or the filling of the thus produced solubilisate into a dispensing cap according to the invention is carried out under a shielding gas. Suitable shielding gases are e.g. carbon dioxide, nitrogen, helium and argon. Preferred are carbon dioxide and nitrogen.

In a further embodiment the solubilisate of the at least one pharmaceutically active agent and/or dietary supplement is packed into the dispensing cap according to the invention under vacuum. Thereby oxygen is removed from the at least one fillable chamber of the dispensing cap in order to avoid oxidation processes.

Confusing and even contradictory definitions of the term "solubilisate" can be found in the art. In order to avoid any ambiguity a solubilisate according to the invention is defined as follows:
A **solubilisate** is the composition of the substance to be solubilized and the solubilizing agents as defined according to the invention, preferred as laid out in the Examples. Further addition of a solvent or diluent shall not be covered by this term. The solubilisate according to the invention is produced first, e.g. by one of the aforementioned solubilization techniques, and then filled into a dispensing cap according to the invention.

In case of a micelle, liposome or cyclodextrin technique the solubilisate according to the invention is characterized by the respective molecular complexes formed by the respective solubilizing agents, and the at least one dietary supplement and/or pharmaceutically active agent solubilized in these respective complexes.

In case of a self-emulsification technique the solubilisate according to the invention is characterized by the substantially complete solubilization of the substance, thus being a nearly perfect solution in which the molecules behave completely as independent entities in a solution and substantially undergo the distribution and thermodynamic rules of Brownian motion. This solution is different from the mixture with the diluent (e.g. water). Said solution (solubilisate) is suitable to be solved in a diluent in a second step. Thus such a solubilisate is a clear solution containing the respective pharmaceutically active agent and/or dietary supplement in a high concentration. When such a solubilisate is diluted in a diluent the respective concentration the respective pharmaceutically active agent and/or dietary supplement diminishes accordingly to the added amount of the diluent.

In general, the solubilisate is not meant for intake without dilution. In most cases, a portioned solubilisate according to the invention accounts to a volume of a few milliliters.

In the scope of this patent application the terms **"solubilization aggregate"** or **"solubilization essence"** shall be used synonymously to "solubilisate".

A solubilisate according to the invention must be differentiated from a **suspension** (colloidal suspension). The term suspension defines a heterogeneous mixture containing solid particles that sooner or later will undergo sedimentation. It is also different from an **emulsion** which is defined as a mixture of two liquids which usually are immiscible.

For increasing the bioavailability of a substance the complete solubilization is highly preferable.

A solubilisate according to the invention must also be differentiated from a **concentrate.** A concentrate is a compound, respectively a composition of compounds without a diluent. Upon release of a concentrate into a diluent the concentrate dissolves itself either completely in the diluent or forms a suspension or emulsion with the diluent. A concentrate does not need the interaction with a solubilizing agent and/or a solvent, as it is intrinsically solvable in water or an aqueous solution. The present invention does not refer to the use of a concentrate in a dispensing cap.

The term solubilisate used according to the invention must be differentiated from the **finished solution,** respectively the drinkable liquid to be imbibed. This finished solution according to the invention is generated by diluting the solubilisate according to the invention in a diluent, preferably an aqueous solution, in order to produce a beverage, respectively fluid dosage form ready for intake by the consumer, respectively the patient.

A **diluent** in the scope of the present application is a diluting agent (dilutant, thinner). It is not part of the solubilisate according to the invention and is not included in a dispensing cap according to the invention.

In the scope of this patent application the term "solubilisate" refers only to the solubilisate intended to be packed or already packed in the dispensing cap according to the invention. It does not refer to the fluid that is generated transitorily during the release process of the solubilisate inside the dispensing cap according to the invention into the "finished solution" inside the beverage container. This transitory fluid shall be referred to as "interim fluid" in the scope of the present patent application. The interim fluid differs from the solubilisate.

In the scope of the present application the term **"solubilizing agent"** refers to any chemical substance that is added to a dietary supplement and/or pharmaceutically active agent in order to solubilize it so that this dietary supplement and/or pharmaceutically active agent can be solved thereupon in an aqueous solution. The term "**solubilizer**" shall be used synonymously.

In alternative terms in the scope of the present application, **"first liquid"** refers to the "solubilisate" and **"second liquid"** to the "finished solution".

In the scope of the present application the term "medicine" shall comprise human and veterinary medicine.

A great advantage of such a solubilisate consists in its small volume. Thus it can be easily portioned to patient- or consumer-friendly units, or relatively huge amounts of a solubilized substance can be shipped at low costs. In order to produce a finished solution the dilution of the solubilisate in an aqueous medium (e.g. tap water or mineral water) can be easily carried out by medical staff, patients or consumers. In another aspect of the invention the dispensing cap according to the invention has the advantage to allow a packaging of oxidation-sensitive, light (incl. UV irradiation)- sensitive, heat-sensitive and/or moisture-sensitive dietary supplements and/or pharmaceutically active agents.

Solubilisates and finished solutions of dietary supplements or pharmaceutically active agents prepared by such a solubilization technique should display a long-term stability so that they offer a reasonable shelf-life. Otherwise they are not very attractive for producers, vendors and finally also for customers or patients. Such a long-term stability is not easy to achieve with many solubilisates, in particular in liquid dosage forms for oral administration. The solubilisates according to the invention, in a dispensing cap according to the invention, as well as the finished solutions to the invention show a very good long-term stability, as can be seen in the Examples.

The internationally accepted BCS (Biopharmaceutical Classification System) classifies drug substances into four classes: Class 1 (high solubility - high permeability), Class 2 (low solubility - high permeability), Class 3 (high solubility - low permeability and Class 4 (low solubility - low permeability).

Herein the term **solubility** refers to the highest dose strength that is subject to an FDA biowaiver request (https://www.fda.gov/OHRMS/DOCKETS/98fr/3657gd3.pdf, as of March 7th, 2017). A drug is classified as highly soluble when the highest dose strength is soluble in 250 ml or less of aqueous media over the pH range of 1 - 7.5. Correspondingly, drug substances that can't be solubilized that way are classified as poorly soluble (= not highly soluble).

Herein the term permeability refers to the extent of absorption of a drug in humans across the intestinal membrane (mucosa). According to the established definition a drug is classified as highly permeable if 90% or more of the orally administered dose are resorbed in the gastrointestinal tract. Correspondingly, a drug having an absorption rate of less than 90% is classified as low permeable.

Thus solubility and permeability are intrinsic substance properties. Resorption and bioavailability, however, describe pharmaceutic parameters that may be improved by suitable measures. While resorption refers to the fraction from the orally applied substance amount that is absorbed from the gastrointestinal tract the bioavailability of a substance depends not only from resorption but also from protein binding in blood and from pharmacokinetic parameters such as first-pass metabolism.

According to the invention in a preferred embodiment pharmaceutical drugs having a poor solubility as defined above are used for the production of a solubilisate.

According to the invention it is preferred that pharmaceutical drugs having a poor permeability as defined above are used for the production of a solubilisate.

According to the invention it is particularly preferred that pharmaceutical drugs having a poor solubility as well as a poor permeability as defined above are used for the production of a solubilisate (Class 4 compounds).

Examples for Class 4 pharmaceutical drugs, without being limiting, are: acetaminophen (paracetamol), acyclovir, azathioprine, azithromycin, calcitriol, carisoprodol, cefdinir, cefixime, cefuroxime axetil, cephalexin, chlorothiazide, chlorthalidone, clarithromycin, cyclosporine, dapsone, dexamethasone, dronabinol, dutasteride, furosemide, glipizide, griseofulvin, hydrochlorothiazide, indinavir sulfate, isradipine, linezolid, loperamide, mebendazole, mercaptopurine, mesalamine, methylprednisolone, modafinil, nabumetone, nelfinavir mesylate, norelgestromin, nystatin, oxcarbazepine, oxycodone HCl, progesterone, pyrimethamine, ritonavir, spironolactone, sulfamethoxazole, trimethoprim, taladafil.

For dietary supplements, the term bioavailability is used slightly differently. In most cases they are consumed orally. Thus this term defines the quantity or fraction of the ingested dose that is absorbed.

According to the invention it is preferred that dietary supplements having a poor bioavailability are used for the production of a solubilisate. It is preferred that their bioavailability is less than 50%, more preferred less than 40%, more preferred less than 30%, even more preferred less than 20%, particularly preferred less than 15% and most preferred less than 10%.

Examples for compounds or plant extracts used as dietary supplements known to have a poor bioavailability are, without being limiting: flavones, flavonols, flavon-3-ols, flavonones, flavonoids, resveratrol, turmeric, curcumin, curcuminoids, demethoxycurcumin, bisdemethoxycurcumin, bis-o-demethyl curcumin, quercetin, ellagic acid, naringenin, betulin, betulinic acid, folic acid (folate), ubiquinone (Q10, coenzyme Q), glutathione, eicosapentaenoic acid (EPA), docosahexaenoic acid (DHA), uridine, chromium dichloride, L-carnitine, ursolic acid, catechin, epicatechin, epigallocatechin (EGC), epigallocatechin gallate (EGCG), epicatechin gallate (ECG), polyphenols, berberin, melatonin, polydatin, isoflavones, liposoluble vitamins A (retinol, retinal), D, E (tocopherols), F, K, α- and β-keto-boswellic acid, L-tryptophan, 5-hydroxytryptophan, L-glycine, inositol, β-carotene, tocotrienols, ascorbyl palmitate, lecithin, lutein, luteolin, lycopene, zeaxanthin, β-cryptoxanthin, red clover, saw palmetto lipid extract, ω-3 fatty acids, steroidal terpenes, non-steroidal terpenes, terpenoids; saponins, sapogenins, diosgenin, *Dioscorea spec.* extract, *Dioscorea villosa* extract, protodioscin, *Tribulus terrestris* extract, essential oils, hypericin, xanthorhizol, pyrogallol, genistein, wogonin, morin, kaempferol, *Bacopa monneri* extract, bacopin, bacoside A, bacoside A3, bacoside B, xanthorhizol, ginseng extract, *Gingko biloba* extract, pycnogenol, capsaicin, *Rubia cordifolia* extract, *Lawsennia iermis* extract, *Aloe vera* extract, piperin, α-lipoic acid, bromelain, phlorizin, crocin, crocetin, bioperine, acerola, proanthocyanidins, anthocyanidins, aglycones of anthocyanins silibinin, silymarin, gingerols, ceramides, isoprene, prenol, isovaleric acid, geranyl pyrophosphate, eucalyptol, limonene, pinene, farnesyl pyrophosphate, artemisinin, bisabolol, geranylgeranyl pyrophosphate, phytol, taxol, forskolin, aphidicolin, squalene, lanosterol, oils, such as shark or other cartilaginous fish oils, vegetable oils, or oils from amaranth seed, rice, wheat germ or olives; squalenes, retinoids, tannins, cinnamic acid, lignins, as well as phytosterols such as β-sitosterol laurate ester, α-sitosterol laurate ester, γ-sitosterol laurate ester, campesterol myristearate ester, stigmasterol oleate ester, campesterol stearate ester, β-sitosterol oleate ester, β-sitosterol palmitate ester, β-sitosterol linoleate ester, α-sitosterol oleate ester, γ-sitosterol oleate ester, β-sitosterol myristearate ester, β-sitosterol ricinoleate ester, campesterol laurate ester, campesterol ricinoleate ester, campesterol oleate ester, campesterol linoleate ester, stigmasterol linoleate ester, stigmasterol laurate ester, stigmasterol caprate ester, α-sitosterol stearate ester, γ-sitosterol stearate ester, α-sitosterol myristearate ester, γ-sitosterol palmitate ester, campesterol ricinoleate ester, stigmasterol ricinoleate ester, campesterol ricinoleate ester, β-sitosterol, α-sitosterol, γ-sitosterol, campesterol, stigmasterol, and stigmasterol stearate ester; extracts from adaptogenic plants such as *Eleutherococcus senticosus* (Siberian ginseng, eleuthero, ciwujia), *Rhodiola rosea* (rose root), *Schisandra chinensis* (five flavor berry), *Panax ginseng* (ginseng), *Gynostemma pentaphyllum* (Jiao Gu Lan), *Morinda citrifolia* (noni, Indian mulberry), *Lentinula edodes* (shiitake), *Ganoderma spec.* (reishi, lingzhi mushroom) such as *Ganoderma lucidum, Ganoderma tsugae* and *Ganoderma sichuanense, Grifola frondosa* (maitake mushroom, hen-of-the-woods), *Agaricus spec.* (almond mushroom) such as *Agaricus subrufescens* and *Agaricus blazei Murill, Withania somnifera* (ashwagandha, winter cherry), *Ocimum tenuiflorum* (tulsi, holy basil), *Lepidum meyenii* (maca), *Andrographis paniculata* (kalmegh), *Cannabis sativa* (marihuana), *Tabebuia impetiginosa* (lapacho), *Astragalus membranaceus* (astragalus, tragacanth).

It is empirically known that poorly water-soluble pharmaceutically active agents or dietary supplements achieve an improved resorption and/or bioavailability upon being solubilized by means of a suitable method. Therefore the present application refers also to a dispensing cap according to the invention, in which the solubilisate of the at least one pharmaceutically active agent and/or dietary supplement enhances the resorption and/or bioavailability of at least one of said pharmaceutically active agents or dietary supplements, in comparison to the non-solubilized substance or substances.

Selected pharmaceutically active agents and/or dietary supplements have been solubilized by means of the aforementioned solubilizing techniques, rendering solubilisates of these substances (see Examples 1 to 12). Thus the present application refers also to a dispensing cap, in which the solubilisate is prepared from a substance selected from a group comprising ß-carotene, coenzyme Q₁₀, piperine, green tea extract, folic acid and quercetin, if the substance is a dietary supplement, or selected from a group comprising furosemide, azithromycin, aciclovir, hydrochlorothiazide, glipizide and clarithromycin, if the substance is a pharmaceutically active agent.

A further aspect of the invention is that some pharmaceutical drugs (medicinal products) or dietary supplements intrinsically have a bitter or unpleasant taste. In case of pharmaceutical drugs this may seriously impair patient compliance, in case of dietary supplements such a taste may be a serious commercialization obstacle. A solubilisate according to the invention can significantly help to mask this bitter or unpleasant taste by caging the substance. Micelle, liposome or self-emulsifying solubilisates use to have a neutral taste, cyclodextrin-based solubilisates a slightly sweetish taste.

Thus the present invention relates also to a dispensing cap containing a solubilisate of at least one pharmaceutical drug or dietary supplement in which a bitter and/or unpleasant taste of the at least one pharmaceutical drug or dietary supplement is masked by the solubilisate. Preferably, this taste-masking solubilisate is prepared by micelle, liposome, self-emulsification or cyclodextrin complexation technology.

This is particularly useful in veterinary medicine when it comes to administer a veterinary drug a bitter or unpleasant taste to an animal in need thereof in an oral dosage form.

Examples of pharmaceutical drugs with a bitter or unpleasant taste comprise, without being limiting, acetaminophen, albuterol, aminoguanidine hydrochloride, aminophylline, amitriptyline, amoxicillin trihydrate, ampicillin, amlodipine besylate, aspirin, azithromycin, barbiturates, berberin chloride, caffeine, calcium carbonate, calcium pantothenate, cephalosporins, cetirizine, chloramphenicol, chlordiazepoxide, chloroquine, chlorpheniramine, chlorpromazine, cimetidine, ciprofloxacin, clarithromycin, codeine, demerol, dextromethorphan, digitoxin, digoxin, diltiazem hydrochloride, diphenhydramine, diphenylhydantoin, doxazosin mesylate, doxylamine succinate, eletriptan, enoxacin, epinephrine, erythromycin, ethylefrine hydrochloride, etinidine, famotidine, fluconazole, glipizide, guaifenesin, ibuprofen, indeloxazine hydrochloride, lidocaine, lomotil, loratadine, lupitidine, magnesium oxide, meclizine, methacholine, morphine, neostigmine, nifentidine, niperotidine, nizatidine, ofloxacin, paracetamol, pefloxacin, penicillin, phenobarbital, phenothiazine, phenylbutazone, phenylpropanolamine, pipemidic acid, pirbuterol hydrochloride, piroxicam, prednisolone, propranolol hydrochloride, pseudoephedrine, pyridonecarboxylic acid antibacterials, ranitidine, roxatidine, salicylic acid, sertraline hydrochloride, sildenafil, spironolactone, sulbactam sodium, sulfonamides, sulfotidine, sulpyrine, sultamicillin tosylate, tenidap, terfenadine, theophylline, trimethoprim, tuvatidine, valdecoxib, zaltidine, and zonisamide.

In a preferred embodiment the solubilisate in the dispensing cap contains a BCS Class 4 pharmaceutical drug with a bitter or unpleasant taste in which said taste can be masked by the solubilisate according to the invention. Suitable examples comprise acetaminophen (paracetamol), azithromycin, clarithromycin, glipizide and trimethoprim.

Many dietary supplements have also a bitter or unpleasant taste, in particular many phytochemicals such as alkaloids, tannins, phenolic or polyphenolic compounds, flavonoids, isoflavones, isoflavone glucosides, glucosinolates, isothiocyanates, cucurbitacins, oxygenated tetracyclic triterpenes.

A further aspect is that some solubilisates may stick to the inner walls of the dispensing cap to a certain degree and are not a 100% released upon preparing a beverage from a dispensing cap according to the invention. This depends mainly on the combination of substances to be solubilized and the used solubilization technique as well as of the material of the dispensing cap and its specific shape. This problem can be overcome or at least widely mitigated by agitating the beverage container with the mounted dispensing cap after triggering the release mechanism. Thus the liquid inside the beverage container is rinsed inside the dispensing cap through its ruptured membrane and solves the solubilisate that is still sticking to the inner walls of the dispensing cap.

In another embodiment a non-stick coating film is applied onto the inner walls of the dispensing cap. This non-stick coating film should be inert, biocompatible and should not disintegrate or detach at common temperatures for transport and storage. Useful examples for such a non-stick coating film are teflons such as PTFE (polytetrafluoroethylene), FEP (fluorinated ethylene propylene copolymer), PFA (perfluoroalkoxy) and ETFE (ethylene tetrafluoroethylene copolymer); anodized aluminium and silicones. Suitable techniques for applying such a non-stick coating film onto the inner walls of dispensing caps include anodizing, dip spinning, cathodic dip painting, nanocoating, wet painting, powder coating, zinc thermal diffusion, polymer coating, thermal spraying, drum spraying, vacuum coating, chrome substitute and plasma deposition. It is among the knowledge of a person skilled in the art to optimize the precise process parameters.

Thus the present application refers also to a dispensing cap suitable for hosting a solubilisate, in which the internal surface of said cap is partially or completely covered with a non-stick coating film.

In yet another embodiment of the invention at least one additional dietary supplement and/or pharmaceutically active agent is provided in the water or aqueous solution with which the beverage container is filled.

The aforementioned solubilisates of pharmaceutical drugs or dietary supplements alone or in combination can be optionally combined with a variety of excipients and/or additives in the dispensing caps according to the invention and/or in the liquid in the beverage container, as laid out in the following. The excipients and/or additives, however, are not part of the solubilisate according to the invention.

Suitable vitamins are for example vitamin C (L-ascorbic acid, sodium L-ascorbate, calcium L-ascorbate, potassium L-ascorbate, L-ascorbyl 6-palmitate), vitamin A (retinol, retinyl acetate, retinyl palmitate, beta-carotene), vitamin D (cholealciferol, ergoalciferol), vitamin E (D-alpha-tocopherol, DL-alpha-tocopherol, D-alpha-tocopheryl acetate, DL-alpha-tocopheryl acetate, D-alpha-tocopheryl succinate), vitamin K (phylloquinone), vitamin B1 (thiamin hydrochloride, thiamin mononitrate), vitamin B2 (riboflavin, sodium riboflavin 5'-phosphate), niacin (nicotinic acid, nicotinamide), pantothenic acid (calcium D-pantothenate, sodium D-pantothenate, D-panthenol), vitamin B6 (pyridoxine hydrochloride, pyridoxine 5'-phosphate), folic acid (pteroyl monoglutaminic acid), vitamin B12 (cyanocobalamine, hydroxocobalamine), biotin (D-biotin).

Suitable minerals to be included are for example calcium (calcium carbonate, calcium chloride, citric acid calcium salt, calcium gluconate, calcium glycerophosphate, calcium lactate, ortho-phosphoric acid calcium salt, calcium hydroxide, calcium oxide), magnesium (magnesium acetate, magnesium carbonate, magnesium chloride, citric acid magnesium salt, magnesium gluconate, magnesium glycerophosphate, ortho-phosphoric acid magnesium salt, magnesium lactate, magnesium hydroxide, magnesium oxide, magnesium sulfate), iron (iron carbonate, iron citrate, iron ammonium citrate, iron gluconate, iron fumarate, iron sodium diphosphate, iron lactate, iron sulfate, iron diphosphate, ferric saccharate, elemental iron), copper (copper carbonate, copper citrate, copper gluconate, copper sulfate, copper lysine complex), iodine (sodium iodide, sodium iodate, potassium iodide, potassium iodate), zinc (zinc acetate, zinc chloride, zinc citrate, zinc gluconate, zinc lactate, zinc oxide, zinc carbonate, zinc sulfate), manganese (manganese carbonate, manganese chloride, manganese citrate, manganese gluconate, manganese glycerophosphate, manganese sulfate), sodium (sodium bicarbonate, sodium carbonate, sodium chloride, sodium citrate, sodium gluconate, sodium lactate, sodium hydroxide, ortho-phosphoric acid sodium salt), potassium (potassium bicarbonate, potassium carbonate, potassium chloride, potassium citrate, potassium gluconate, potassium glycerophosphate, potassium lactate, potassium hydroxide, ortho-phosphoric acid potassium salt), selenium (sodium selenite, sodium hydrogen selenite, sodium selenite), chrome (chrome-(III)-chloride, chrome-(III)-sulfate), molybdenum (ammonium molybdate (molybdenum (VI), sodium molybdate (molybdenum (VI)), fluorine (sodium fluoride, potassium fluoride), chlorine, phosphor.

Trace elements are dietary minerals that are needed by the organism in very small amounts for growth, development and physiology, for example as co-enzymes. Some of them are virtually always present in the organism in sufficient quantities, others have to be substituted in persons in need thereof. They can be selected from the group comprising chrome, cobalt, iron, iodine, copper, manganese, molybdenum, selenium, zinc, fluoride, silicon, arsenic, nickel, rubidium, tin, vanadium. They can be substituted either as a pure element or in any of the mineral forms mentioned above.

Stimulants are often and worldwide used in drinks. According to the World Health Organization (WHO) this term refers to any kind of substances increasing, accelerating or improving neuronal activity. These substances have often a psychomimetic effect. Most popular stimulants include xanthines such as caffeine, theophylline and theobromine. Guaraná contains the aforementioned xanthines. A further popular stimulant is nicotine, respectively nicotinic acid. However, there is a broad group of stimulants that in many countries are banned by law, expected to be banned in the near future, or underlie a strict regulation of health authorities, needing the prescription of a physician. This is due to their dependence potential and other hazards to consumers' health, attention deficits in traffic etc., or negative effects on social life. Thus group includes a.o. amphetamine and its derivatives, a group of piperazine derivatives, cocaine and drugs for the treatment of narcolepsy and attention deficit hyperactivity disorder (ADHD). Hence the use of this group of substances according to the invention may be possible too. Preferred is the use of caffeine.

Suitable antioxidants can be selected from the group comprising lactic acid, ascorbic acid, sodium ascorbate, calcium ascorbate, potassium ascorbate, fatty acid esters of ascorbic acid, ascorbyl palmitate, ascorbyl stearate, tocopherols, alpha-tocopherol, gamma-tocopherol, delta-tocopherol, propyl gallate, octyl gallate, dodecyl gallate, ethyl gallate, guaiac resin, erythorbic acid, sodium erythorbate, erythorbin acid, sodium erythorbin, tert-butylhydroquinone, butylated hydroxyanisole, butylated hydroxytoluene, mono-, di-, trisodium phosphate, mono-, di-, tripotassium phosphate, anoxomer, ethoxyquin, potassium lactate, stannous chloride, sodium thiosulfate, 4-hexylresorcinol, glucose oxidase.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminium potassium sulfate, aluminium ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Often pharmaceutically active agents or dietary supplements are provided as a salt. For pharmaceutically active agents the pharmaceutically acceptable salts are listed in the respective pharmacopoeias. Thus they can be selected from the group comprising as cationic salts the respective sodium, potassium, calcium, lithium, magnesium salts, as anionic salts the respective chloride, bromide, sulfate, phosphate, acetate, citrate, oxalate, malonate, salicylate, p-aminosalicylate, malate, fumarate, succinate, ascorbate, maleate, sulfonate, phosphonate, perchlorate, nitrate, formate, propionate, gluconate, digluconate, lactate, tartrate, hydroxy maleate, pyruvate, phenyl acetate, benzoate, p-aminobenzoate, p-hydroxybenzoate, dinitrobenzoate, chlorbenzoate, mesylate, ethanesulfonate, nitrite, isethionate, ethylene sulfonate, tosylate, naphthyl sulfonate, 4-amino-besylate, camphorsulfonate, alginate, caprate, hippurate, pectinate, phthalate, quinate, mandelate, o-methyl mandelate, hydrogen besylate, picrate, cyclopentanepropionate, D-o-toluyl tartrate, tartronate, besylate, alpha-methyl benzoate, (o, m, p-)methyl benzoate, naphthylamine sulfonate, cinnamate, acrylate, trifluoroacetate, isobutyrate, phenyl butyrate, heptanoate, xylyl sulfonate, adipate, aspartate, bisulfate, borate, butyrate, camphorate, dodecylsulfate, laurate, glucoheptonate, glycerylphosphate, hemisulfate, hexanoate, hydroiodide, 2-hydroxy-ethanesulfonate, lactobionate, lauryl sulfate, nicotinate, oleate, palmitate, pamoate, persulfate, 3-phenyl propionate, pivalate, stearate, thiocyanate, undecanoate, valerate, and glycolate. It is understood that these salts can also be used in preparations of dietary supplements used in the present invention.

In general, salts prepared from an acid with a low pKₐ display a good solubility in an aqueous medium. For such salts the use of a solubilization technique as defined above is not mandatory. In the dissociated state these salts may even interfere with some of said solubilization techniques. Therefore the use of salts generated from an acid with a pKₐ > 2.0 are preferred, more preferred a pKₐ > 3.0, still more preferred a pKₐ > 4.0 and most preferred a pKₐ > 5.0.

The term "pharmaceutical excipients" refers to natural or synthetic compounds that are added to a pharmaceutical formulation alongside the pharmaceutical active agent. They may help to bulk up the formulation, to enhance the desired pharmacokinetic properties or the stability of the formulation, as well as be beneficial in the manufacturing process. Advantageous classes of excipients according to the invention include without being limiting, flavors, colorants, preservatives, sweeteners, carriers, additional solubilizing agents, buffers, preservatives, opacifiers.

It can be advantageous, respectively mandatory to add one or more pharmaceutically acceptable carrier to a pharmaceutically active agent. Eligible are all carriers known in the art and combinations thereof. For liquid dosage forms and emulsions suitable carriers are for example additional solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethyl glycols, fatty acid esters of sorbitan.

In some embodiments it may be desirable that in the finished solution some foam is generated. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some solubilisates according to the invention may appear slightly foamy upon preparation. Though this does not interfere with the desired application it may affect patient compliance in case of a medication or the commercial success in case of dietary supplements. Therefore it may be desirable to add a pharmaceutically or nutritionally acceptable anti-foaming agent (defoamer) to the solubilisate. Examples are polydimethylsiloxane or silicone oil in dietary supplements or simethicone in pharmaceuticals.

Thus the present application refers also to a dispensing cap, in which a nutritionally and/or pharmaceutically acceptable antifoaming agent is added to the at least one pharmaceutically active agent and/or dietary supplement.

Colorants are excipients that bestow a colorization to the composition of the drink, respectively the dosage form. These excipients can be food colorants. They can be adsorbed on a suitable adsorption means such as clay or aluminium oxide. The amount of the colorant may vary between 0.01 and 10 % per weight of the finished solution, preferred between 0.05 and 6 % per weight, more preferred between 0.1 and 4 % per weight, most preferred between 0.1 and 1 % per weight.

Suitable food colorants are curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulphite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminium, silver, gold, pigment rubine, tannin, orcein, ferrous gluconate, ferrous lactate.

Flavor enhancers are widely used for food and drinks. Suitable examples are glutamic acid, monosodium glutamate, monopotassium glutamate, calcium diglutamate, monoammonium glutamate, magnesium diglutamate, guanylic acid, sodium guanylate, disodium guanylate, dipotassium guanylate, calcium guanylate, inosinic acid, disodium inosinate, dipotassium inosinate, calcium inosinate, calcium 5'-ribonucleotides, disodium 5'-ribonucleotides, glycine, sodium glycinate, zinc acetate, gum benzoic, thaumatin, glycyrrhizin, neohesperidine dihydrochalcone, glyceryl monoacetate, glyceryl diacetate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)mehylaminol] propane), ethylene diamine, ACES (2-[(amino-2-oxoethyl)amino]ethanesulfonic acid), imidazol, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazol, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholino-propanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid dosage forms or supplements can be used on demand. They may be selected from the group comprising sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulphite, sodium bisulphite, sodium metabisulphite, potassium metabisulphite, potassium sulphite, calcium sulphite, calcium hydrogen sulphite, potassium hydrogen sulphite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

Additional emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, crosscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch.

Preferred are glycerin monooleate and stearic acid.

Stabilizers are substances that can be added to prevent unwanted changes. Though stabilizers are not real emulsifiers they may also contribute to the stability of emulsions, respectively solubilisates. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Suitable as additional surface-active solubilizing agents (solubilizers) are for example diethylene glycol monoethyl ester, polyethyl propylene glycol co-polymers, cyclodextrins such as α- and β-cyclodextrin, glyceryl monostearates such as Solutol HS 15 (Macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates etc.

Suitable aromatic and flavoring substances (flavors) comprise above all essential oil that can be used for this purpose. In general, this term refers to volatile extracts from plants or parts of plants with the respective characteristic smell. They can be extracted from plants or parts of plants by steam distillation.

Examples are: Essential oils, respectively aromatic substances from sage, cloves, chamomile, anise, star anise, thyme, tea tree, peppermint, mint oil, menthol, cineol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight of the finished solution, preferred 0.001 to 6% per weight, more preferred 0.001 to 4% per weight, most preferred 0.01 to 1% per weight. Application- or single case-related it may be advantageous to use differing quantities.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

Opacifiers are substances that render the drinkable liquid opaque, if desired. They must have a refractive index substantially different from the solution, in most cases here water. At the same time they should be inert to the other components of the composition. Suitable examples include titanium dioxide, talc, calcium carbonate, behenic acid, cetyl alcohol, or mixtures thereof.

According to the invention all of the aforementioned excipients and classes of excipients can be used without limitation alone or in any conceivable combination thereof, as long as the inventive use of a solubilisate is not thwarted, toxic actions may occur or the respective national legislations are infracted.

Thus the present application refers also to a dispensing cap, wherein at least one from a group of non-solubilized substances comprising vitamin, mineral, trace element, stimulant, dietary supplement, herbal product, adaptogen, antioxidant, colorant, flavoring substance, flavor enhancer, aromatic substance, sweetener, isotonizing agent, foaming agent, pharmaceutically active agent, pharmaceutical excipient, acidifier, acidity regulator, buffer, preservative, stabilizer, pH regulator, and opacifier is loaded additionally to said solubilisate into the at least one filling chamber of said dispensing cap.

In a further aspect the present patent application refers also to a beverage system comprising a dispensing cap and a beverage container.

The beverage container must be suitable for the storage of a drinkable liquid over a certain period of time. Thus the material of the beverage container material must be sufficiently durable and the set-up of the beverage container preferably inherently stable. Further, it must provide a reasonable volume for storing a suitable liquid volume corresponding to the targeted purpose of the drinking liquid.

Thus said beverage container can be selected from a group comprising bottles, flasks, vials, flacons, ampules, beverage cartons, Tetra Pak^{®}, cans, canteens, mugs, steins, pouches, stand-up pouches, barrels, kegs, hose-shaped containers. Preferred containers are bottles, vials and beverage cartons.

A prerequisite is that said beverage container has an outlet, respectively an opening or recess through which the solubilisate according to the invention can be released into the drinking liquid inside the container. The dispensing cap has to be mounted over this opening or recess. This can be done in a screw cap manner with an interlocking thread, as known from many bottle or beverage container types, or by a clamp mechanism, a folding mechanism, a crimp mechanism, a swing stopper mechanism, a flip-flop cap, a sealing tape. Preferred are screw caps.

In yet another embodiment of the invention at least one excipient and/or additive, as listed above, is provided in the water or aqueous solution with which the beverage container is filled.

The present patent application discloses also said beverage system for use as a dosage form in medicine, wherein said solubilisate is prepared from at least one pharmaceutically active agent.

In yet another aspect the present patent application refers to a method for providing said beverage system, comprising the following steps:
a) Providing a beverage container as defined above;
b) providing a dispensing cap as defined above;
c) producing a solubilisate of at least one pharmaceutically active agent and/or dietary supplement by means of a solubilization technology as defined above;
d) filling the solubilisate resulting from step c) into the at least one fillable chamber of the dispensing cap;
e) tightly closing the at least one chamber of the dispensing cap by means of a suitably configured sealing membrane or by assembling the respective parts of the dispensing cap;
f) optionally, filling a drinkable liquid into the beverage container; and
g) mounting the thus filled dispensing cap onto the beverage container.

In yet another aspect the present patent application refers also to a finished solution produced by
a) providing a beverage system as defined above, wherein said beverage container is pre-filled with a drinkable liquid,
b) triggering said release mechanism of said dispensing cap as defined above for releasing said solubilisate into the drinkable liquid, and
c) optionally, agitating the beverage system for a better mixing of the drinkable liquid and the released solubilisate.

The present patent application refers also to said finished solution for use in medicine, wherein the solubilisate is produced from at least one pharmaceutically active agent.

### Examples

In all ensuing examples the dissolution step of the solubilisate was carried out three times in order to generate three finished solutions from each solubilisate stock solution, respectively. Throughout all examples the respective results for the three finished solutions were consistent.

### Example 1:

In order to generate a solubilisate of the loop diuretic furosemide (a BCS class IV pharmaceutical drug) 4.5 g *Poloxamer* 188 and 1.25 g α-tocopherol were heated up to 60°C until they melt. 4.76 ml aqua bidest (25%) were heated to 60°C and used to cover the molten mixture. It was waited until a gel was formed. In a second solution 14.29 ml aqua bidest (75%) were provided and 0.2 g furosemide were added under stirring. Then this second solution was added to the gel (for the method cf. WO 2007/104173 A2). Thus a solubilisate is yielded which is apt to form stable micelles enclosing the compound to be solubilized. As the indicated amount of aqua bidest is needed therefor it has to be regarded as a solubilisate and not as a concentrate. This solubilisate (25 ml) had approximatively the following composition:

| | |
|---|---|
| *Poloxamer 188* | 18 wt% |
| α-tocopherol | 5 wt% |
| furosemide | 0.8 wt% |
| aqua bidest | 76.2 wt% |

5 ml of this solubilisate were filled into the bottom part of an INCAP dispensing cap. The upper part of the INCAP was put thereon and the two parts were tightly closed by pressing down the upper part. The thus charged cap was mounted on a 500 ml PET bottle having a standard screw thread (28 mm, right-turning) and filled with still mineral water. Releasing the solubilisate into the still mineral water and agitating the whole beverage system yielded a beverage containing 40 mg furosemide, a standard dosage of this drug. The experiment was repeated two times (n = 3). Each time a clear solution without any slurs was generated. No compound sedimented, flocculated or re-crystallized during a two hours observation period.

### Example 2:

In order to generate a solubilisate of the very lipophilic dietary supplement β-carotene (a precursor of vitamin A, a terpenoid contained in a variety of plants such as carrots, pumpkins etc.) 4.5 g *Poloxamer* 188 and 1.25 g α-tocopherol were heated up to 60°C until they melt, as in Example 1. 4.81 ml aqua bidest (25%) were heated to 60°C and used to cover the molten mixture. It was waited until a gel was formed. In a second solution 14.41 ml aqua bidest (75 %) were provided and 30 mg β-carotene were added under stirring. Then this second solution was added to the gel. Thus a solubilisate is yielded which is apt to form stable micelles enclosing the compound to be solubilized when added to any quantity of water. This solubilisate (25 ml) had approximatively the following composition:

| | |
|---|---|
| *Poloxamer 188* | 18 wt% |
| α-tocopherol | 5 wt% |
| β-carotene | 0.3 wt% |
| aqua bidest | 76.7 wt% |

This solubilisate was used for filling a Fusion Cap with 2 ml. A finished solution was prepared, as described in Experiment 1 (n = 3). This yielded a beverage containing 6 mg β-carotene, a recommended daily dosage for this dietary supplement. Each time a clear mildly orange-colored solution without any slurs was generated. No compound sedimented, flocculated or re-crystallized upon cooling down of the beverage to room temperature during a four hours observation period.

### Example 3:

Azithromycin is a broad-spectrum antibiotic that is widely used against infections by some Gram-positive, some Gram-negative and many atypical bacteria. Azithromycin belongs to BCS Class 4 (low solubility - low permeability) pharmaceuticals.

### Preparation of the solubilisate:

The following indications refer to the weight percent of the mixture. A solubilisate of ca. 20 ml was generated. Azithromycin was provided, and then the solubilizing agents were admixed one by one under stirring for 5 min at room temperature (20 ± 5°C) and atmospheric pressure.

| | |
|---|---|
| azithromycin | 25.0 % |
| 1-palmitoyl-2-oleoyl-sn-glycero-3-phosphocholine (POPC) | 34.0 % |
| MCT oil | 31.7 % |
| mixture of 2-lysophosphatidylcholine and 2-lysophosphatidylcholine (1:1) | 3.2 % |
| ethanol | 3.0 % |
| oleic acid | 1.1 % |
| glyceryl stearate | 1.7 % |
| glyceryl oleate | 0.2 % |
| beta-tocopherol | 0.1 % |

Then the composition was cautiously heated under continued stirring, with an approximate temperature increment of 2°C / min. After ca. 8 min (ca. 36°C) the composition started to become a clear solution. This solubilization process lasted for ca. 7 min more. Thus a solubilisate according to the invention was obtained after ca. 15 min at ca. 50°C. Then the heating and the stirring was stopped and the resulting solubilisate was allowed to cool down to room temperature.

2 ml of this solubilisate were filled in the dome-shaped chamber of a VizCap dispensing cap, the lid was tightly closed and the cap was mounted on a 500 ml PET bottle having a standard screw thread (28 mm, right-turning) and filled with still mineral water. A finished solution was prepared by releasing the solubilisate into the mineral water (n = 3). The finished solution became quickly clear and had a slightly whitish appearance. This rendered a beverage containing 500 mg azithromycin, a typically recommended daily dosage.

The bitter taste of azithromycin - which often causes a compliance problem, especially with children - could be covered by this solubilisate.

### Example 4:

Aciclovir is an antiviral pharmaceutical agent. It is frequently used in the treatment of *Herpes simplex* infections, shingles and chickenpox. Aciclovir belongs to BCS Class 4 (low solubility - low permeability) pharmaceuticals.

### Preparation of the solubilisate:

The following indications refer to the weight percent of the mixture. A solubilisate of ca. 20 ml was generated. Aciclovirwas provided, and then the solubilizing agents were admixed one by one under stirring for 5 min at room temperature (20 ± 5°C) and atmospheric pressure.

| | |
|---|---|
| aciclovir | 4.0 % |
| dimyristoyl phosphatidylcholine (DPMC) | 57.0 % |
| MCT oil | 30.1 % |
| 1-lysophosphatidylcholine and 2-lysophosphatidylcholine (weight ratio: 1:1 | 3.0 % |
| ethanol | 2.5 % |
| oleic acid | 1.2 % |
| glyceryl stearate | 1.9 % |
| glyceryl oleate | 0.15 % |
| alpha-tocopherol | 0.05 % |

Then the composition was cautiously heated under continued stirring, with an approximate temperature increment of 1°C / min. After ca. 45 min (ca. 65°C) the composition started to become a clear solution. This solubilization process lasted for ca. 5 min more. Thus a solubilisate according to the invention was obtained after ca. 50 min at ca. 70°C. Then the heating and the stirring was stopped and the resulting solubilisate was allowed to cool down to room temperature.

5 ml of this solubilisate were filled into the bottom part of an INCAP dispensing cap. The upper part of the INCAP was put thereon and the two parts were tightly closed by pressing down the upper part. The thus charged cap was mounted on a 500 ml PET bottle having a standard screw thread (28 mm, right-turning) and filled with still mineral water. Releasing the solubilisate into the still mineral water and agitating the whole beverage system yielded a beverage with a slightly whitish appearance containing 200 mg aciclovir. This corresponds to a standard tablet dosage of this drug. The experiment was repeated two times (n = 3). Each time a clear solution without any slurs was generated. No compound sedimented, flocculated or re-crystallized during an eight hours observation period.

The taste of aciclovir - which patients often describe as unpleasant, sometimes also as metallic - could be covered by this solubilisate.

### Example 5:

Hydrochlorothiazide is a diuretic pharmaceutical agent. It is frequently used in the treatment of high blood pressure, swelling due to fluid build-up, diabetes insipidus, renal tubular acidosis and in the prophylaxis of persons with an elevated risk of kidneys stones. Hydrochlorothiazide belongs to BCS Class 4 (low solubility - low permeability) pharmaceuticals.

### Preparation of the solubilisate:

The following indications refer to the weight percent of the mixture. A solubilisate of ca. 20 ml was generated. Hydrochlorothiazide was provided, and then the solubilizing agents were admixed one by one under stirring for 5 min at room temperature (20 ± 5°C) and atmospheric pressure.

| | |
|---|---|
| hydrochlorothiazide | 5.0 % |
| non-hydrogenated soy bean PC and POPC (weight ratio: 1:1) | 50.0 % |
| MCT oil | 36.2 % |
| L-alpha-lysophosphatidylcholine | 2.6 % |
| ethanol | 2.2 % |
| oleic acid | 1.8 % |
| glyceryl stearate | 1.9 % |
| glyceryl oleate | 0.2 % |
| ascorbyl palmitate | 0.1 % |

Then the composition was cautiously heated under continued stirring, with an approximate temperature increment of 1 °C / min. After ca. 38 min (ca. 58°C) the composition started to become a clear solution. This solubilization process lasted for ca. 7 min more. Thus a solubilisate according to the invention was obtained after ca. 45 min at ca. 65°C. Then the heating and the stirring was stopped and the resulting solubilisate was allowed to cool down to room temperature.

5 ml of this solubilisate were filled into the cavity of a 28 mm ViCap dispensing cap and the dispensing cap assembled. The thus charged cap was mounted on a 500 ml PET bottle having a standard screw thread (28 mm, right-turning) and filled with still mineral water. Releasing the solubilisate into the still mineral water and agitating the whole beverage system yielded a beverage with a slightly whitish appearance containing 25 mg hydrochlorothiazide, a standard tablet dosage. The experiment was repeated two times (n = 3). Each time a clear solution without any slurs was generated. No compound sedimented, flocculated or re-crystallized during an four hours observation period.

The taste of hydrochlorothiazide - which patients often describe as metallic - could be covered by this solubilisate.

### Example 6:

Coenzyme Q₁₀ (synonyms: ubiquinone, ubidecarone, coenzyme Q, CoQ₁₀) is a ubiquitous coenzyme in most animals. Three redox states of coenzyme Q₁₀ have been described. The molecule acts as a 2 electron carrier and a one electron carrier, corresponding to its role in the electron transport chain and as a radical scavenger. Coenzyme Q₁₀ is hardly soluble in an aqueous environment and poorly absorbed in the body. However, it is a broadly marketed dietary supplement.

### Preparation of the solubilisate:

The following indications refer to the weight percent of the mixture. A solubilisate of ca. 20 ml was generated. Coenzyme Q₁₀ was provided, and then the solubilizing agents were admixed one by one under stirring for 5 min at room temperature (20 ± 5°C) and atmospheric pressure.

| | |
|---|---|
| coenzyme Q₁₀ | 8.0 % |
| non-hydrogenated soy bean phosphatidylcholine | 26.0 % |
| MCT oil | 58.85 % |
| 2-lysophosphatidylcholine | 2.8 % |
| ethanol | 1.8 % |
| oleic acid | 0.9 % |
| glyceryl stearate | 1.4 % |
| glyceryl oleate | 0.2 % |
| alpha-tocopherol | 0.05 % |

Then the composition was cautiously heated under continued stirring, with an approximate temperature increment of 0.5°C / min. After ca. 36 min (ca. 38°C) the composition started to become a clear solution. This solubilization process lasted for ca. 12 min more. Thus a solubilisate according to the invention was obtained after ca. 48 min at ca. 44°C. Then the heating and the stirring was stopped and the resulting solubilisate was allowed to cool down to room temperature.

5 ml of this solubilisate were filled into the bottom part of an INCAP dispensing cap. The upper part of the INCAP was put thereon and the two parts were tightly closed by pressing down the upper part. The thus charged cap was mounted on a 500 ml PET bottle having a standard screw thread (28 mm, right-turning) and filled with still mineral water. Releasing the solubilisate into the still mineral water and agitating the whole beverage system yielded a beverage with a milky white yellowish containing 400 mg coenzyme Q₁₀. This corresponds to an often recommended daily dosage of this dietary supplement. The experiment was repeated two times (n = 3). Each time a clear solution without any slurs was generated. No compound sedimented, flocculated or re-crystallized during a two hours observation period.

### Example 7:

Piperine (IUPAC name: 1-[5-(1,3-benzodioxol-5-yl)-1-oxo-2,4-pentadienyl]piperidine) is the main alkaloid from *Piper negrum* (black pepper) and usually won by alcoholic extraction. It is a colorless to yellow solid at room temperature and poorly water-soluble. As many spicy substances piperine stimulates metabolism and gastrointestinal secretion, and it displays antimicrobial actions. Furthermore, it is a bioavailability enhancer. It was found to inhibit human CYP3A4 and P-glycoprotein, two enzymes involved in first-pass metabolism of xenobiotics. Thus, it can be used as a dietary supplement and/or as a bioavailability enhancer of other substances (mainly other dietary supplements).

### Preparation of the solubilisate:

The following indications refer to the weight percent of the mixture. A solubilisate of ca. 10 ml was generated. Piperine was provided, and then the solubilizing agents were admixed one by one under stirring for 5 min at room temperature (20 ± 5°C) and atmospheric pressure.

| | |
|---|---|
| piperine | 5.0 % |
| 2-dioleyl-SN-glycero-3-phosphocholine (DOPC) | 31.0 % |
| MCT oil | 55.25 % |
| 2-lysophosphatidylcholine | 3.0 % |
| ethanol | 2.7 % |
| oleic acid | 1.1 % |
| glyceryl stearate | 1.7 % |
| glyceryl oleate | 0.15 % |
| beta-tocopherol | 0.1 % |

Then the composition was cautiously heated under continued stirring, with an approximate temperature increment of 1.5°C / min. After ca. 27 min (ca. 60°C) the composition started to become a clear solution. This solubilization process lasted for ca. 16 min more. Thus a solubilisate according to the invention was obtained after ca. 43 min at ca. 84°C. Then the heating and the stirring was stopped and the resulting solubilisate was allowed to cool down to room temperature.

2 ml of this solubilisate were used for filling a Fusion Cap. A finished solution was prepared, as described in Experiment 1 (n = 3). This yielded a beverage containing 10 mg piperine, a dosage inside the margin for a recommended daily dosage for this dietary supplement (5 - 15 mg/d). Under stirring the finished solution became quickly clear and had a pale white appearance. No compound sedimented, flocculated or re-crystallized upon cooling down of the beverage to room temperature during a four hours observation period.

The characteristic poignant taste (more accurately, odor) of piperine could be covered by this solubilisate.

### Example 8:

Green tea extract is produced from green tea leaves (Camellia sinensis). The main components are green tea catechins, such as epigallocatechin-3-gallate (EGCG), epicatechin (EC), epicatechin-3-gallate (ECg), epigallocatechin (EGC), catechin, and gallocatechin (GC), with EGCG being the most abundant of them in green tea extract. Green tea extract is often used as a dietary supplement, aiming at healthy effects attributed to catechins. They include above all antioxidant, anticarcinogenic, anti-inflammatory and anti-radiation actions. However, catechins, in particular EGCG, show a poor bioavailability and the solubility in water is rather limited.

### Preparation of the solubilisate:

The following indications refer to the weight percent of the mixture. A solubilisate of ca. 20 ml was generated. Green tea extract (Sabinsa, Langen, Germany) was provided, and then the solubilizing agents were admixed one by one under stirring for 5 min at room temperature (20 ± 5°C) and atmospheric pressure.

| | |
|---|---|
| green tea extract | 10.0 % |
| phosphatidylcholine (PC and DMPC, weight ratio 1:1) | 30.0 % |
| MCT oil | 52.0 % |
| 2-lysophosphatidylcholine | 2.8 % |
| ethanol | 2.5 % |
| oleic acid | 0.8 % |
| glyceryl stearate | 1.6 % |
| glyceryl oleate | 0.2 % |
| delta-tocopherol | 0.1 % |

Then the composition was cautiously heated under continued stirring, with an approximate temperature increment of 2.5°C / min. After ca. 33 min (ca. 102°C) the composition started to become a clear solution. This solubilization process lasted for ca. 4 min more. Thus a solubilisate according to the invention was obtained after ca. 37 min at ca. 112°C. Then the heating and the stirring was stopped and the resulting solubilisate was allowed to cool down to room temperature.

5 ml of this solubilisate were filled into the bottom part of an INCAP dispensing cap. The upper part of the INCAP was put thereon and the two parts were tightly closed by pressing down the upper part. The thus charged cap was mounted on a 500 ml PET bottle having a standard screw thread (28 mm, right-turning) and filled with still mineral water. Releasing the solubilisate into the still mineral water and agitating the whole beverage system yielded a beverage containing 500 mg green tea extract. Green tea extract contains ca. 50 weight-% of EGCG, with a variation range. Thus the beverage contains ca. 250 mg EGCG which is a recommended daily dosage of this dietary supplement. The experiment was repeated two times (n = 3). under stirring the finished solution became quickly clear and had a pale white brownish appearance. Each time a clear solution without any slurs was generated. No compound sedimented, flocculated or re-crystallized during a two hours observation period.

The grassy (herbal) taste of diluted green tea extract could be covered by this solubilisate. This also holds true for the sometimes bitter taste of green tea, depending on the blend, which renders green tea unpopular for some people.

### Example 9:

25 ml of a liposome-based solubilisate of the sulfonylurea anti-diabetic drug glipizide (a BCS class IV pharmaceutical drug) were generated. The liposomes were produced from the phospholipid 1,2-dimyristoyl-sn-glycero-3-phosphocholine (DPMC). The phospholipid was solubilized by dissolving 50 mg DPMC in 2.5 ml t-butanol and heating the mixture in a 37°C water bath for 5 minutes. Then the solution was stored at -20°C in a tight container protected from light exposure. 50 mg glipizide were dissolved in 500 µl DMSO and also stored at -20°C in a tight container protected from light exposure. The next day the solutions were thawed and 2.5 ml of the DPMC solution, 500 µl of the glipizide solution and 22 ml t-butanol were thoroughly mixed (for liposome technology cf. US 2008/0103213 A1). This solubilisate had approximatively the following composition:

| | |
|---|---|
| glipizide | 0.2 wt% |
| DPMC | 0.2 wt% |
| DMSO | 2.0 wt% |
| t-butanol | 97.6 wt% |

5 ml of this solubilisate were filled into the cavity of a 28 mm ViCap dispensing cap and the dispensing cap assembled. The dispensing cap was mounted on a 500 ml PET bottle having a standard screw thread (28 mm, right-turning) filled with sparkling mineral water, the release mechanism triggered and the resulting finished solution gently agitated. This yielded a beverage containing 10 mg glipizide, a standard dosage of this drug. The experiment was repeated two times (n = 3). Each time a clear solution without any slurs was generated. No compound sedimented, flocculated or re-crystallized during an four hours observation period. The solution was clear, slightly yellow and had a neutral acceptable taste.

### Example 10:

25 ml of a liposome-based solubilisate of folic acid (vitamin B₉) were generated in analogy to Example 5. The liposomes were produced from the phospholipid 1,2-dimyristoyl-sn-glycero-3-phosphocholine (DPMC). The phospholipid was solubilized by dissolving 50 mg DPMC in 2.5 ml t-butanol and heating the mixture in a 37°C water bath for 5 minutes. Then the solution was stored at -20°C in a tight container protected from light exposure. 2 mg folic acid were dissolved in 500 µl DMSO and also stored at -20°C in a tight container protected from light exposure. The next day the solutions were thawed and 2.5 ml of the DPMC solution, 500 µl of the folic acid solution and 22 ml t-butanol were thoroughly mixed. This solubilisate had approximatively the following composition:

| | |
|---|---|
| folic acid | 0.008 wt% |
| DPMC | 0.2 wt% |
| DMSO | 2.0 wt% |
| t-butanol | 97.8 wt% |

This solubilisate was used for filling a ViCap^{®} dispensing cap with 5 ml. The dispensing cap was mounted on a 500 ml PET bottle filled with sparkling mineral water, the release mechanism triggered and the resulting finished solution gently agitated. This yielded a beverage containing 400 µg folic acid, a recommended daily dose for this dietary supplement. The experiment was repeated two times (n = 3). The solution was clear, slightly yellow and had a neutral acceptable taste.

### Example 11:

In order to generate a solubilisate of the antibiotic clarithromycin (a BCS class IV pharmaceutical drug) a 10 ml composition was generated. First, clarithromycin (2.5 g), β-cyclodextrin (70 mg) and fumaric acid (500 mg) were micronized and then dispersed together in a dispersion of the HPC in ware (7% w/w). A small amount of simethicone emulsion was added to defoam the dispersion. A blend of the non-ionic polymer hydroxypropylcellulose (630 mg) and unmicronized β-cyclodextrin (100 mg) was prepared in a planetary mixer. The clarithromycin dispersion was added to it and then the entire mixture is passed through an extruder. The resulting material was dried at 45°C. The dried exudate was milled again. Then it was dispersed in 6.2 ml aqua bidest. (for the method cf. US 2003/0091627 A1).

This solubilisate had approximatively the following composition:

| | |
|---|---|
| clarithromycin | 25 wt% |
| β-cyclodextrin | 1.7 wt% |
| fumaric acid | 5 wt% |
| hydroxypropylcellulose | 6.3 wt% |
| aqua bidest | 62 wt% |

This solubilisate was used for filling a VizCap dispensing cap with 2 ml and preparing a finished solution, as described before (n = 3). This yielded a beverage containing 500 mg clarithromycin, a standard dosage for antibiotic therapy. Each time a solution without any visible particles was generated. As many macrolide antibiotics, clarithromycin has a bitter taste. In the present formulation this taste is masked and the beverage had a slightly sweetish taste.

### Example 12:

In order to generate a solubilisate of the bioflavonoid quercetin for intake as a dietary supplement a 20 ml composition was generated. Similar to Example 11, quercetin (4 g), β-cyclodextrin (140 mg) and fumaric acid (1 g) were micronized first and then dispersed together in a dispersion of the HPC in ware (7% w/w). A small amount of simethicone emulsion was added to defoam the dispersion. A blend of the non-ionic polymer hydroxypropylcellulose (1.26 g) and unmicronized β-cyclodextrin (200 mg) was prepared in a planetary mixer. The quercetin dispersion was added to it and then the entire mixture is passed through an extruder. The resulting material was dried at 45°C. The dried exudate was milled again. Then it was dispersed in 12.4 ml aqua bidest.

This solubilisate had approximatively the following composition:

| | |
|---|---|
| quercetin | 20.0 wt% |
| β-cyclodextrin | 1.7 wt% |
| fumaric acid | 5 wt% |
| hydroxypropylcellulose | 6.3 wt% |
| aqua bidest | 67 wt% |

5 ml of this solubilisate were filled into the bottom part of an INCAP dispensing cap. The upper part of the INCAP was put thereon and the two parts were tightly closed by pressing down the upper part. The thus charged cap was mounted on a 500 ml PET bottle having a standard screw thread (28 mm, right-turning) and filled with still mineral water. Releasing the solubilisate into the still mineral water and agitating the whole beverage system yielded a beverage containing 1 g quercetin, a recommended daily dosage. The experiment was repeated two times (n = 3). Each time a solution without any visible particles was generated. No compound sedimented, flocculated or re-crystallized during a two hours observation period.

The bitter taste, typical for polyphenolic flavonoids such as quercetin was covered. The beverage has a slightly sweetish taste.

## Claims

1. A dispensing cap with at least one fillable chamber containing a solubilisate of at least one pharmaceutically active agent and/or dietary supplement,
wherein the dispensing cap is suitably configured to be mounted on an outlet of a beverage container and said solubilisate can be released into the beverage container by operating a releasing mechanism of the dispensing cap.

2. The dispensing cap according to claim 1, wherein said solubilisate is prepared from at least one poorly water-soluble substance or extract.

3. The dispensing cap according to any of claims 1 or 2, wherein said solubilisate is prepared by means of micelle, liposome, self-emulsification or cyclodextrin complexation technology.

4. The dispensing cap according to any of claims 1 to 3, wherein said dispensing cap is selected from a group comprising ViCap^{®}, VizCap, Activate^{®} cap, Optima functional cap, BioGaia Cap, Cedevita cap, INCAP, PowerCap^{®}, Yoli Blast Cap, Mojo organics cap, Karma cap^{®}, Tap-the-Cap^{®}, Delo Cap^{®}, Aspin^{®} Dispensing Bottle Cap, CapStaticX, Berocca Twist 'N' Go^{®}, Fusion Cap.

5. The dispensing cap according to any of claims 1 to 4, wherein the at least one chamber of the dispensing cap is oxygen- and/or moisture-restricted and/or the processing of said solubilisate and/or the loading of the at least one fillable chamber of said dispensing cap with said solubilisate is carried out under a shielding gas or vacuum.

6. The dispensing cap according to any of claims 1 to 5, wherein the solubilisate is prepared from a substance selected from the group comprising furosemide, glipizide, clarithromycin, azithromycin, aciclovir and hydrochlorothiazide, if the substance is a pharmaceutically active agent, or selected from the group comprising β-carotene, folic acid, quercetin, coenzyme Q₁₀, piperine and green tea extract, if the substance is a dietary supplement.

7. The dispensing cap according to any of claims 1 to 6, wherein said solubilisate provides an enhanced resorption and/or bioavailability of at least one of the solubilized pharmaceutically active agents and/or dietary supplements in a human, in comparison to the non-solubilized substance or substances.

8. The dispensing cap according to any of claims 1 to 7, wherein said solubilisate provides a masking of a bitter and/or unpleasant taste of at least one pharmaceutically active agent and/or dietary supplement solubilized therein.

9. The dispensing cap according to any of claims 1 to 8, wherein at least one from a group of non-solubilized substances comprising vitamin, mineral, trace element, stimulant, dietary supplement, herbal product, adaptogen, antioxidant, colorant, flavoring substance, flavor enhancer, aromatic substance, sweetener, isotonizing agent, foaming agent, pharmaceutically active agent, pharmaceutical excipient, acidifier, acidity regulator, buffer, preservative, stabilizer, pH regulator, and opacifier is loaded additionally to said solubilisate into the at least one filling chamber of said dispensing cap.

10. A beverage system, comprising a dispensing cap as defined in claim 1 and a beverage container.

11. The beverage system according to claim 10, wherein the beverage container is selected from a group comprising bottles, flasks, vials, flacons, ampules, beverage cartons, Tetra Pak^{®}, cans, canteens, mugs, steins, pouches, stand-up pouches, barrels, kegs, hose-shaped containers.

12. The beverage system according to any of claims 10 or 11 for use as a dosage form in medicine, wherein said solubilisate is prepared from at least one pharmaceutically active agent.

13. Method for providing a beverage system as defined in any of claims 10 to 12, comprising the following steps:
a) Providing a beverage container as defined in claim 11;
b) providing a dispensing cap as defined in claim 4;
c) producing a solubilisate of at least one pharmaceutically active agent and/or dietary supplement by means of a solubilization technology as defined in claim 3;
d) filling the solubilisate resulting from step c) into the at least one fillable chamber of the dispensing cap;
e) tightly closing the at least one chamber of the dispensing cap by means of a suitably configured sealing membrane or by assembling the respective parts of the dispensing cap;
f) optionally, filling a drinkable liquid into the beverage container; and
g) mounting the thus filled dispensing cap onto the beverage container.

14. A finished solution produced by
a) providing a beverage system as defined in any of claims 10 to 12, wherein said beverage container is pre-filled with a drinkable liquid,
b) triggering said release mechanism of said dispensing cap as defined in claim 1 for releasing said solubilisate into the drinkable liquid, and
c) optionally, agitating the beverage system for a better mixing of the drinkable liquid and the released solubilisate.

15. A finished solution according to claim 14 for use in medicine, wherein the solubilisate is produced from at least one pharmaceutically active agent.
